# EUROPEAN PATENT APPLICATION

(11) **EP 1 227 322 A1**
(43) Date of publication of application: **31.07.2002**
(21) Application number: 02250425.2
(22) Date of filing: 22.01.2002
(51) Int. Cl.: G01N 33/573, C12Q 1/25

(54) **Methods of detecting poly(ADP-ribose) polymerase enzymatic activity**

(30) Priority: 30.01.2001 US 265135 P
(71) Applicant: Pfizer Products Inc., Groton, Connecticut 06340 (US)
(72) Inventor: Brown, Janice Ann, Pfizer Global Research and, Groton, Connecticut 06340 (US); Marala, Ravi Balaya, Pfizer Global Research and, Groton, Connecticut 06340 (US)
(74) Representative: Hayles, James Richard

(57) **Abstract**

The invention provides methods for assaying poly(ADP-ribose) polymerase (PARP) activity and methods for the identification of modulators of PARP activity. These methods comprise contacting immobilized PARP with NAD under conditions that allow PARP auto-ribosylation, contacting the auto-ribosylated PARP with a detectable marker to form a complex and then indirectly or directly measuring the amount of detectable marker bound to the auto-ribosylated PARP, this amount being indicative of PARP activity.

## Description

### FIELD OF THE INVENTION

The invention relates to the fields of pharmacology and medicine. More specifically, the invention provides methods to assay poly(ADP-ribose) polymerase (PARP) activity and to identify a modulator of PARP activity.

### BACKGROUND

PARP is a zinc-finger DNA-binding protein that catalyzes the transfer of the ADP-ribose moiety from nicotinamide-adenine-dinucleotide (NAD) to itself (auto-ribosylation) and to a limited number of protein acceptors involved in chromatin architecture and metabolism. PARP appears to function in response to transient and localized DNA strand breaks in cells, which may arise through a variety of processes including DNA repair, replication, recombination, and gene rearrangement (see, Alkhatib *et al*. (1987) *Proc. Natl. Acad. Sci.* (USA) **84**:1224-1228).

PARP nuclear protein is present at a density of up to one PARP molecule per 10-20 nucleosomes. The enzyme is activated 10-20 fold by DNA damage, transferring 50-100 ADP-ribose moieties to each acceptor site of target proteins. PARP converts NAD into nicotinamide and polymers of ADP-ribose at glutamic acid residues of target nuclear proteins. The majority of ADP-ribose polymer chains are linked to PARP, itself, through auto-ribosylation.

Over-activation of PARP as a result of substantial DNA damage can markedly deplete cells of NAD and ATP; for each ADP-ribose unit transferred by PARP, one molecule of NAD is consumed and an equivalent of four molecules of ATP are required to regenerate NAD from nicotinamide (Gaal, *et al.* (1987) *Trends in Biological Sciences* **12**:129; Berger, N. A. (1985) *Rad. Res.* **101**:4). This rapid depletion of NAD and ATP in injured or stressed cells may contribute to cell death.

Inhibitors of PARP have been shown to reduce myocardial infarct size in animal models, to help prevent ischemic injury in the brain (Plaschke *et al.* (2000) *Neurosci. Lett.* **284:**109-112), to increase apoptosis in cancer cells (Kaufmann, *et al.* (1993) *Can. Res.* **53:**3976-3986), to prevent infiltration of neutrophils and subsequent inflammation (Jijon *et al.* (2000) *Am. J. Physiol.* **279**:G641-G651), and to suppress the production of nitric oxide synthase in macrophages (Messmer *et al.* (1996) *Febs. Lett.* **384:**162-166 and Mohr et *al.* (1998) *Proc. Natl. Acad. Sci.* **95**:5045-5050). Thus, there is a need for fast, sensitive and reliable methods for measuring PARP activity, particularly in high throughput formats, to identify agonists and antagonists of PARP activity.

Assays for PARP activity are known in the art, but these assays are not well suited to high throughput analysis due to their use of radioactive isotopes, unreliable antibodies, laborious procedures and/or due to their inadequate sensitivity. For example, Trevigen (Gaithersburg, MD) offers for sale a PARP assay kit (Trevigen Catalogue # 4667-50-K) that requires the use of ³²P- or ³H-labeled NAD and a trichloroacetic acid (TCA) precipitation step, and a PARP assay kit (Trevigen Catalogue # 4669-96-K) that monitors ribosylation of histones, a PARP substrate; Cheung *et al.* (2000) *(Anal. Biochem.* **282:**24-28) describe a scintillation proximity assay for PARP, and Decker *et al* (1999) *(Clin. Cancer Res.* **65:**1169-1172) describe an ELISA, nonradioactive, colorimetric PARP assay.

Thus, there remains a need in the art for a simplified, sensitive PARP assay that is easily adapted to a high throughput format and does not create radioactive waste.

### Summary of the Invention

The present invention features methods to assay PARP activity and methods to identify modulators of PARP activity. These methods are fast, reliable, sensitive, and do not generate radioactive waste. Moreover, the methods of the present invention meet the needs of the pharmaceutical industry for high throughput analysis.

In one aspect, the invention provides a method to assay PARP activity. The method involves contacting an immobilized PARP with NAD under conditions that allow PARP auto-ribosylation. The NAD utilized by the method is biotinylated or avidin-conjugated. Next, the auto-ribosylated PARP is contacted with a detectable marker that is avidin-conjugated in the case where the NAD of the method is biotinylated, or contacted with a biotinylated detectable marker in the case where the NAD of the method is avidin-conjugated, thereby forming a complex between the auto-ribosylated PARP and the detectable marker. The amount of detectable marker complexed to PARP is then measured as it is indicative of the amount of PARP activity present.

In preferred embodiments, the NAD is biotinylated and the detectable marker is avidin-conjugated, the avidin-conjugated detectable marker is alkaline phosphatase, the PARP is immobilized on a multiwell plate, and/or the assay method is conducted at 4°C.

In another aspect, the invention provides a method to identify a modulator of PARP activity. The method involves contacting an immobilized PARP with NAD in the presence of a test agent under conditions that allow PARP autoribosylation. The NAD utilized by the method is biotinylated or avidin-conjugated. Next, the auto-ribosylated PARP is contacted with a biotinylated detectable marker if the NAD utilized is avidin-conjugated. Alternatively, the autoribosylated PARP is contacted with an avidin-conjugated detectable marker if the NAD utilized is biotinylated. The amount of detectable marker is measured and compared to the amount of detectable marker in a control reaction executed without the test agent. An altered amount of detectable marker in the presence of the test agent relative to the amount of detectable marker found in the control reaction indicates that the test agent is a modulator of PARP. In preferred embodiments, the NAD is biotinylated and the detectable marker is avidin-conjugated, the avidin-conjugated detectable marker is alkaline phosphatase, the PARP is immobilized on a multiwell plate, and/or the assay method is conducted at 4°C.

In certain embodiments of the methods disclosed herein, the present invention also includes the use of other molecules besides biotin and avidin for the identical purpose of incorporating a detectable marker into a complex with the auto-ribosylated PARP.

In another aspect, the invention provides a kit comprising PARP immobilized on a solid-support, *e.g.*, a multiwell plate, biotinylated NAD or avidin-conjugated NAD, and an avidin-conjugated detectable marker in the case where the NAD of the method is biotinylated, or a biotinylated detectable marker in the case where the NAD of (b) is avidin-conjugated.

As used herein, the term "PARP" refers to any PARP-1 polypeptide, a zinc finger protein that catalyzes the transfer of the ADP-ribose moiety from nicotinamide-adenine-dinucleotide)(NAD) to itself (auto-ribosylation) and to other protein acceptors (see, *e.g.,* GenBank Accession Numbers NM 001618 (human), NM 007415 and AF126717 (mouse), and AJ131705 (*Arabidopsis thaliana*). The PARP used in the methods of the invention may be natural PARP or non-natural PARP. As used herein, the phrase "natural" PARP refers to PARP made by a cell without human intervention. As used herein, the phrase "non-natural" PARP refers to PARP made through standard techniques in recombinant DNA or biochemical technologies. Preferably, human PARP-1 is used (Trevigen, Catalog # 4667-250-01).

By "conditions that allow PARP auto-ribosylation" is meant standard buffer conditions and reagents that enable the auto-ribosylation of PARP, as described herein and in the literature (*see*, *e.g.*, Decker *et al*. (1999) *Clin. Cancer Res.* **5:**1169-72; Hauschildt *et al*.(1992) **288:**255-60; Zhang *et al.,* WO 00/39104; and Webber et *al.,* WO 00/42040).

The term "biotin" is meant to include biotin and any related molecules that specifically bind to an avidin molecule. A "biotinylated NAD" as used herein refers to 6-biotin-17-nicotinamide-adenine-dinucleotide or any other NAD molecule to which biotin has been attached at an alternative site on the ADP-ribose moiety so long as the biotin attachment does not prevent PARP from utilizing the biotinylated NAD in auto-ribosylation. A "biotinylated detectable marker" refers to a detectable marker with an attached biotin, so long as the biotinylation does not prevent detection of the marker.

The term "avidin" is meant to encompass molecules such as avidin, streptavidin, and any other related molecules that specifically bind to a biotin molecule and may be attached (i.e., conjugated) to the ADP-ribose moiety of NAD or to a detectable marker. An "avidin-conjugated NAD" contains avidin attached to the ADP-ribose moiety of NAD so long as the attachment does not prevent PARP from using the NAD in auto-ribosylation. Similarly, an "avidin-conjugated detectable marker" contains avidin attached to the marker at a site that does not prevent detection of the marker.

As used herein, the term "detectable marker" is meant to include any molecule or compound that can be attached to biotin or avidin, or equivalent molecules, and that is quantifiable by any number of methods known in the art such as by measuring optical density, luminescence, or fluorescence. A detectable marker includes any marker that can be quantified directly (*e.g*., fluorescent or luminescent markers), as well as enzymes that can be quantified indirectly by measuring the production of detectable products of the enzyme's reaction (*e.g*., by assessing the formation of reaction products by measuring optical density, luminescence, or fluorescence). Examples of luminescent markers include luminol and acridone; examples of fluorescent markers include fluorescein-5-isothiocyanate (FITC), Cy3, rhodamine, R-phycoerythrin, and tetramethylrhodamine isothiocyanate (TRITC). Detectable markers that are enzymes (and their substrates) include alkaline phosphatase (p-nitrophenyl phosphate (pNPP) and CDP Star™), peroxidase (o-phenylenediamine dihydrochloride (OPD) and 3,3',5,5'-tetramethylbenzidine (TMB)), and β-galactosidase (5-bromo-4-chloro-3-indolyl beta-D-galactopyranoside (X-gal)). These fluorescent, luminescent, and enzymatic markers (including avidin-conjugated markers) and the above-mentioned substrates are available commercially (*e*.*g*., Sigma Aldrich, St. Louis, MO).

The term "detectable marker" may also include all other molecules used for the identical purpose of detecting the auto-ribosylated PARP.

By an "immobilized PARP" is meant a PARP that is attached to a solid support or carrier such that the attachment does not prevent PARP autoribosylation. Attachment may be through covalent linkage or non-covalent linkage (*e.g*., ionic interaction with the support). The PARP molecule may be directly attached to the support or attached indirectly by interaction with an anti-PARP antibody that is itself attached to the support. Exemplary methods for immobilizing PARP are described herein. Solid supports or carriers that can be used include columns, beads, filters, or plates. Preferably, the support is a multi-well plate (*e.g.*, 96- or 384-well) useful for high throughput screening.

As used herein, the term "modulator" refers to a molecule or a compound that either increases or decreases PARP activity. In one embodiment, the modulator is an agonist of PARP activity and the effect is an increase in PARP activity. In another embodiment, the modulator is an antagonist of PARP activity and the effect is a decrease in PARP activity. Such a molecule or compound may also be referred to as "an inhibitor of PARP activity".

It is to be understood that the foregoing general description and the following detailed description are exemplary and explanatory, and are intended to provide further explanation of the invention claimed.

### Description of the Drawings

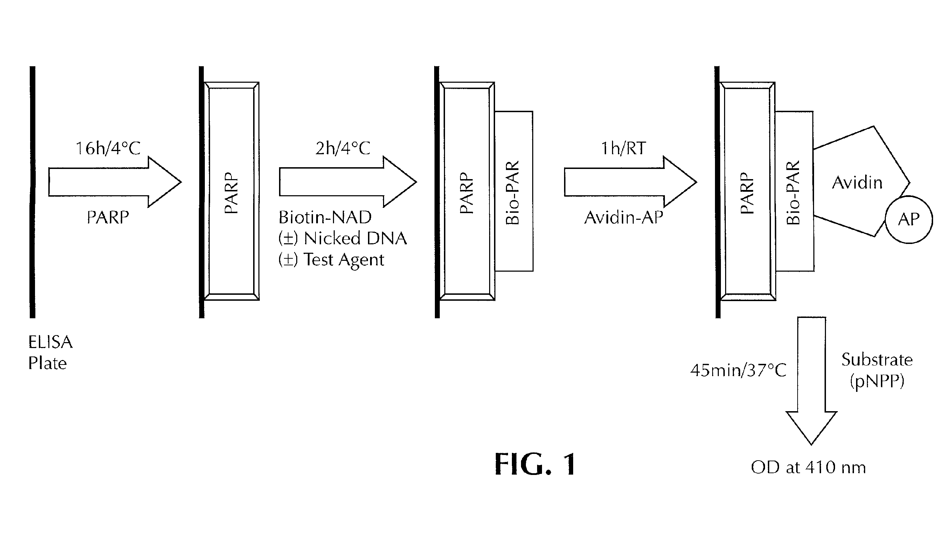

Figure 1 is a diagrammatic representation of one embodiment of the PARP assay methodologies, in which the following abbreviations are used. "PARP" refers to poly(ADP-ribose) polymerase; "pNPP" refers to p-nitrophenyl phosphate; "AP" refers to alkaline phosphatase; "NAD" refers to nicotinamide-adenine-dinucleotide; "ELISA" refers to enzyme linked immunosorbent assay; Bio-PAR refers to biotin-poly(ADP-ribose); and "OD" refers to optical density.

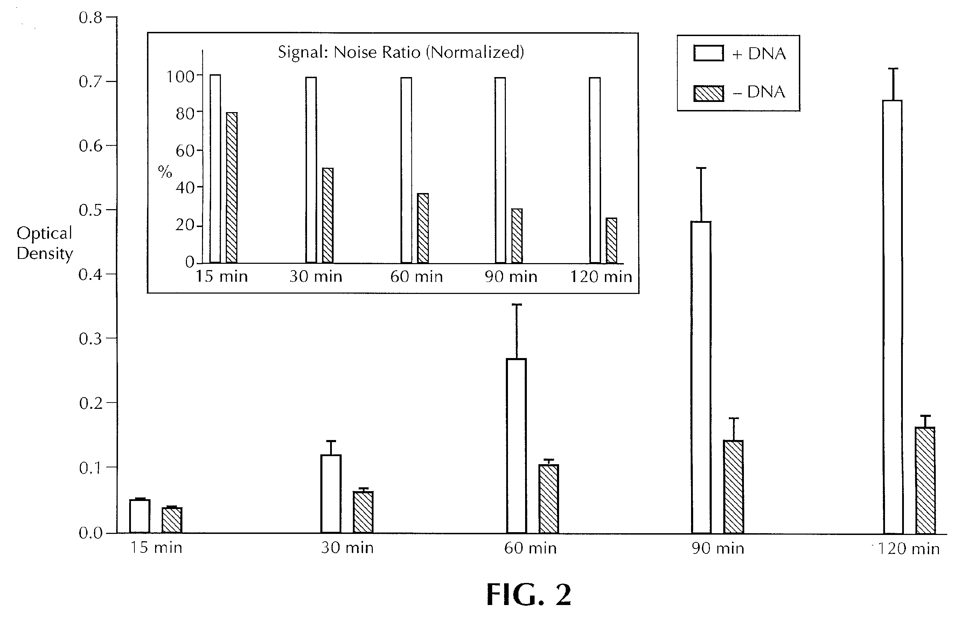

Figure 2 is a graphic representation of a time course at 4°C for the biotinylated NAD PARP assay with and without added sheared DNA. Results with samples containing sheared DNA reflect PARP activity, since the activity of the enzyme is increased in the presence of sheared DNA. The inset graphic representation displays the results of the experiment after each test sample with sheared DNA has been normalized to 100%, thereby depicting the relative contribution of background signal (*i.e.*, signal obtained without added DNA present in the reaction) at each time point.

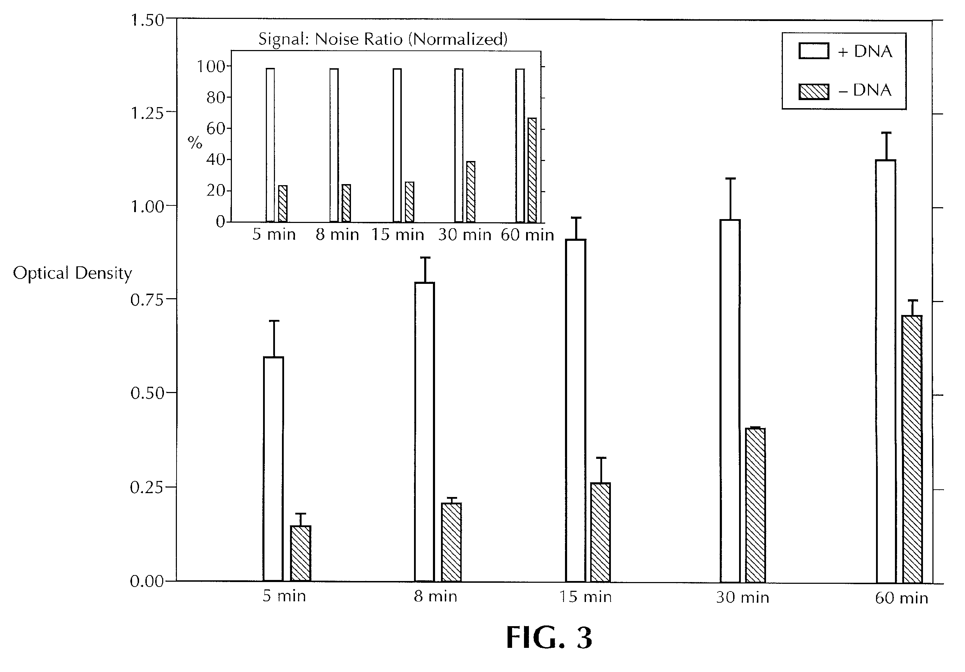

Figure 3 is a graphic representation of a time course at room temperature for the biotinylated NAD PARP assay with and without added sheared DNA. Results with samples containing sheared DNA reflect PARP activity, since the activity of the enzyme is increased in the presence of sheared DNA. The inset graphic representation displays the results of the experiment after each test sample with sheared DNA has been normalized to 100%, thereby depicting the relative contribution of background signal (*i.e.*, signal obtained without added DNA present in the reaction) at each time point.

### Detailed Description

The patent applications, patents, and literature references cited herein indicate the knowledge of those of ordinary skill in this field and are hereby incorporated by reference in their entirety. In the case of inconsistencies between any reference cited herein and the specific teachings of the present disclosure, this disclosure will prevail. Similarly, any inconsistencies between an art-understood meaning of a term and a meaning of a term as specifically taught in the present disclosure will be resolved in favor of this disclosure.

Aspects of the invention utilize techniques and methods common to the fields of molecular biology, cell biology and immunology. Useful laboratory references for these types of methodologies are readily available to those skilled in the art. See, for example, Molecular Cloning, A Laboratory Manual, 2nd. edition, edited by Sambrook, J., Fritsch, B. F. and Maniatis, T., (1989), Cold Spring Harbor Laboratory Press; Current Protocols In Molecular Biology and Current Protocols in Immunology, Wiley Interscience, New York; Harlow & Lane, Antibodies: A Laboratory Manual, Cold Spring Harbor Press, Cold Spring Harbor, N.Y. (1988).

The present invention provides methods to assay PARP activity and methods to identify modulators of PARP activity. Modulators of PARP are useful pharmacological agents in situations involving DNA damage and/or oxidative stress, for example in the treatment of ischemic events, such as myocardial infarction and stroke, and in the treatment of cancer, autoimmune disorders, infections, aging, inflammation, and radiation or chemical exposure.

PARP activity is assayed by first contacting an immobilized PARP with a biotinylated NAD molecule (Trevigen, Gaithersburg, MD), which results in PARP being autoribosylated and, therefore, labeled with biotin. The biotin-labeled PARP is then contacted with avidin that is conjugated to a detectable marker. By measuring the amount of avidin-conjugated detectable marker bound to PARP, one obtains a measurement of autoribosylated PARP, and, therefore, an index of PARP activity.

Alternatively, the assay is conducted with the reverse scheme in which the NAD is avidin-conjugated and the detectable marker is biotinylated.

According to the invention, PARP is immobilized on a solid support. Immobilization can be accomplished using any standard technique, provided that PARP autoribosylation remains detectable. The PARP may be attached directly to the support, or indirectly attached through binding to an anti-PARP antibody that is attached to the support. Exemplary supports are as follows: columns, beads, filters, or plates. Preferably, the support is a multi-well plate (*e.g*., 96-well or 384-well) that is adaptable to a high throughput format assay (*e.g.*, high affinity binding EIA/RIA flat bottom plates, CoStar, Corning, NY; medium or low specific binding plates, CoStar; and polyvinylchloride plates, Falcon, Becton Dickenson, Bedford, MA).

To attach the PARP directly to a support, such as a multi-well plate, PARP can be suspended at a final concentration of 5 µg/ml in immobilization assay buffer (*e.g.*, 50 mM Tris-HCI, pH 8.0, 20 µM ZnCl₂, and 4 mM MgCl₂) which is then applied to each well of the plate (*e*.*g*., 200 µl) and incubated (*e.g.*, approximately 5 hours at room temperature or overnight at 4°C). Such PARP coated supports can be stored for up to 60 hours at 4°C without significant loss of activity, or up to two weeks at -20°C without losing more than 50% activity.

Alternatively, the PARP can be attached to the support indirectly via interaction with an anti-PARP antibody (*e.g.,* Trevigen, Cat. #4338-MC-50) which is attached to the support, such as a 96-well plate (high affinity binding EIA/RIA flat bottom plate, CoStar, Corning, NY). For example, the antibody is diluted (1:600) in immobilization assay buffer and 200 µl is added to each well and incubated for approximately 5-6 hours at room temperature. Following three washes in phosphate buffered saline (PBS), pH 7.0, with 0.05% Tween-20 (Sigma-Aldrich, St. Louis, MO) (PBS-T), 200 µl of PBS containing 5% bovine serum albumin is added to each well and incubated for about 1 hour at 37°C. Following three more washes in PBS-T, a 1 µg/ml solution of PARP enzyme in iced immobilization buffer is added to each well and incubated overnight at 4°C. Prior to use, the supports are washed (*e.g*., three times in PBS-T) to remove unattached PARP and unattached anti-PARP antibody.

Although the present invention is described with respect to using biotinylated NAD and an avidin-conjugated detectable marker, it will be understood by those skilled in the art that the assay methods of the present invention also encompass the use of NAD that is avidin-conjugated and a detectable marker that is biotinylated.

The immobilized PARP is exposed to conditions that allow for autoribosylation. For example, when using 96 well plates, 100-200 µl of autoribosylation assay buffer (*e*.*g*., 50 mM Tris-HCI, pH 8.0, 20 µM ZnCl₂, 4 mM MgCl₂, 1 mM dithiothreitol (DTT), 25 µM biotinylated NAD (Trevigen), sheared DNA (*e.g*., 12.5 µg/ml) (Trevigen), and excess unlabeled NAD (*e.g.*, 100 µM) (Boehringer Mannheim, Indianapolis, IN) is added to each well (preferably, all reagents are at 4°C and plates are on ice) and incubated for an appropriate time as determined by temperature. When incubating at 4°C, the preferred temperature, appropriate incubation times range from about 30 to about 120 min., preferably 90-120 min. When incubating at room temperature, appropriate incubations times range from about 5 to about 30 min., preferably 5-15 min. Control samples lack sheared DNA. Unless otherwise indicated, reagents are available from Sigma-Aldrich (St. Louis, MO). Other standard conditions and autoribosylation assay buffers that may be used are described in the literature (*see, e.g.*, Decker *et al.* (1999) *Clin. Cancer Res.* **5**: 1169-1172; Hauschildt *et al*. (1992) 288: 255-60; Zhang *et al.,* WO 00/39104; and Webber *et al.,* WO 00/42040).

Following incubation, the plates (or another solid support) are washed, and an avidin conjugated detectable marker is added to each well at an appropriate dilution. A preferred marker is avidin-conjugated alkaline phosphatase (Sigma-Aldrich) applied according to manufacturer's recommendations at a 1:70,000 dilution in PBS-T. Following a one hour incubation with plates covered for one hour at room temperature (according to manufacturer's recommendations), the plates are washed three times in PBS-T and blotted dry. When using alkaline phosphatase as the detectable marker, the substrate pNPP (Sigma-Aldrich) is used according to manufacturer's recommendations to generate the detectable product that is measured as an indication of the amount of alkaline phosphatase present in the sample. For example, the pNPP substrate solution is prepared by mixing pNPP with glycine buffer (100 µM glycine, 1 mM MgCl₂, 1 mM ZnCl₂, pH 10.4) for a final pNPP concentration of 1 mg/ml. Following the administration of the pNPP substrate solution (*e.g.,* 200 µl/well), the plates are incubated for about 45 min. at 37°C. Optical density is then determined using a spectrophotometer designed to read the plates at 410 nm.

As one skilled in the art will understand, a detectable marker includes any marker that can be quantified directly (*e.g.*, fluorescent or luminescent markers), as well as enzymes that can be quantified indirectly by measuring the production of detectable products of the enzyme's reaction (*e.g.*, by measuring optical density). Non-limiting examples of luminescent markers include luminol and acridone, and non-limiting examples of fluorescent markers include fluorescein-5-isothiocyanate (FITC), Cy3, rhodamine, R-phycoerythrin, and tetramethylrhodamine isothiocyanate (TRITC). Detectable markers that are enzymes (and their substrates) include alkaline phosphatase (p-nitrophenyl phosphate (pNPP) and CDP Star™), peroxidase (o-phenylenediamine dihydrochloride (OPD) and 3,3',5,5'-tetramethylbenzidine (TMB)), and β-galactosidase (5-bromo-4-chloro-3-indolyl beta-D-galactopyranoside (X-gal)). These fluorescent, luminescent, and enzymatic markers (including avidin-conjugated markers) and the above-mentioned substrates are available commercially (*e.g.*, Sigma Aldrich, St. Louis, MO).

Those skilled in the art will understand that when β-galactosidase is used as the selectable marker, the substrate X-gal is used to produce a detectable product used to quantitate the amount of (3-galactosidase present in the sample. Similarly, when peroxidase is used as the detectable marker, OPD or TMB is used as the substrate to generate the detectable product. Both the avidin-conjugated markers and their respective substrates are commercially available (*e.g.*, from Sigma-Aldrich) and can be used according to manufacturer's recommendations. Optical density is the standard method of quantitating (β-galactosidase or peroxidase activity in a sample using the above-mentioned substrates.

Those skilled in the art will also understand that, if the avidin-conjugated detectable marker is an avidin-conjugated chemiluminescent label or avidin-conjugated fluorescent label, this type of label may be directly detected by standard methods used to measure chemiluminescent or fluorescent properties of the marker.

The present invention includes all means known or that will be developed to measure either the detectable marker or the detectable product of the methods of the invention. The specific device used or mode of taking the measurement is not important as long as it is the appropriate means of detecting and measuring the detectable marker or detectable product.

Using any of the above standard detection schemes, PARP autoribosylation, and, therefore, PARP activity is correlated with the increase in optical density, luminescence, or fluorescence, or other detectable means, after subtracting out the values for control samples lacking sheared DNA.

The PARP activity assays of the present invention can also be used to identify modulators of PARP activity. Such modulators are useful for the purpose of either increasing or decreasing PARP activity. By way of non-limiting example, a modulator of PARP that decreases the activity of PARP is useful for limiting cell death and tissue damage, since decreasing PARP activity will result in the preservation of a cell's energy supply and, thereby, limit cell death and tissue damage.

Examples of agents that are screened include, but are not limited to, nucleic acids (*e.g*., DNA and RNA), carbohydrates, lipids, proteins, peptides, peptidomimetics, small molecules and other compounds. Agents can be selected individually for testing or as part of a library. These libraries are obtained using any of the numerous approaches in combinatorial library methods known in the art, and include: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (*e.g.*, Lam (1997) *Anticancer Drug Des.* **12**:145; U.S. Patent No. 5,738,996; and U.S. Patent No. 5,807,683).

Examples of methods for the synthesis of molecular libraries can be found in the art, for example, in DeWitt *et al.* (1993) *Proc. Natl. Acad. Sci. USA* **90:**6909; Erb *et al.* (1994) *Proc. Natl. Acad. Sci. USA* **91:**11422; Zuckermann *et al.* (1994) *J. Med. Chem.* **37:**2678; Cho *et al.* (1993) *Science* **261**:1303; Carrell *et al.* (1994) *Angew. Chem. Int. Ed. Engl.* **33:**2059; Carell *et al.* (1994) *Angew. Chem. Int. Ed. Engl.* **33:**2061; and Gallop *et al.* (1994) *J*. *Med. Chem.* **37:**1233.

Individual agents or libraries of agents may be presented in solution (*e.g.*, Houghten (1992) *Bio*/*Techniques* **13**:412-421) or on beads (Lam (1991) *Nature* **354:**82-84), chips (Fodor (1993) *Nature* **364:**555-556), bacteria (U.S. Patent No. 5,223,409), spores (U.S. Patent Nos. 5,571,698; 5,403,484; and 5,223,409), plasmids (Cull *et al.* (1992) *Proc. Natl. Acad. Sci. USA* **89:**1865-1869), or phage (Scott (1990) *Science* **249:**386-390; Devlin (1990) *Science* **249:**404-406; Cwirla *et al.* (1990) *Proc. Natl. Acad. Sci. USA* **87**:6378-6382, and Felici (1991) *J. Mol. Biol.* **222:**301-310).

Preferably, the agents are diluted in dimethyl sulfoxide (DMSO) to appropriate concentrations, and included in the auto-ribosylation assay buffer that is applied to PARP-attached supports.

The method of identifying a modulator of PARP activity may be executed with a kit of the invention. As presently demonstrated, PARP may be stably bound to a carrier or solid support such as a 96-well plate for an extended period of time. Such a finding is useful for the purposes of providing a PARP modulator assay kit, which comprises PARP bound to a carrier such as a 96-well plate. The kit optionally contains the appropriately labeled NAD molecule, such as biotin or avidin-labeled NAD, an appropriately conjugated detectable marker, such as an avidin-conjugated detectable marker, and, if appropriate, a specific substrate for the conjugated detectable marker.

The following descriptions illustrate examples of making and practicing the present invention but are not meant to limit the scope of the invention since alternative methods may be utilized to obtain similar results.

### Example 1: Time Course for the PARP Assay

A schematic of the PARP assay provided by the present invention is shown in Figure 1. ELISA plates (96-well) were coated with recombinant human PARP-1 overnight at 4°C.

A partially purified human PARP-1 protein (Trevigen, Catalog #4667-250-01) was coated onto 96-well high affinity binding EIA/RIA flat bottom plate (Costar, Corning, NY) by placing 200 µl of a PARP solution (5.3 µg/ml of PARP in assay buffer (50 mM Tris-HC1, pH 8.0, 20 µM ZnCl₂ and 4 mM MgCl₂)) into each well and incubating the plate overnight at 4°C. Prior to the activity assay, the plates were washed three times in PBS-T.

The PARP reaction(s) was carried out at 4°C for two hours in a total volume of 200 µl of autoribosylation buffer containing 50 mM Tris-HCI, pH 8.0, 20 µM ZnCl₂, 4 mM MgCl₂, 1 mM DTT (Sigma Aldrich, Catalog #D-9779), 25 µM biotinylated NAD (Trevigen), 100 µM unlabelled NAD (Boehringer Mannhein, Catalog #127965), and 12.5 µg/ml sheared DNA (Trevigen). The control reactions were carried out in the absence of sheared DNA in order to determine background activity for the reaction(s). The results of assays carried out with incubations at room temperature and at 4°C are presented in Figures 2 and 3, respectively. Wells were washed three times with PBS-T followed by incubation for 1 hour at room temperature with avidin-alkaline phosphatase (1:70,000) (Sigma-Aldrich) diluted in PBS-T. Plates were washed and incubated for 45 min. at 37°C with substrate (1 mg/ml pNPP) (Sigma-Aldrich) diluted in glycine buffer (100 µM glycine, 1 mM MgCl₂, 1 mM ZnCl₂, pH 10.4). The optical density for each sample was measured at 410 nm.

### Example 2: The PARP Assay Detects Inhibition by Known PARP Inhibitors

The present method was conducted in the presence of known PARP inhibitors of varying potencies, tested at a range of concentrations. The experiment was conducted to determine if the IC₅₀ values generated by the assay of the present invention were in agreement with previously reported IC₅₀ values for these compounds and IC₅₀ values generated by a commercially available [³²P] NAD-based PARP assay (R&D Systems, Inc., Minneapolis, MN, Cat#TA5334). [³²P]-NAD was obtained from Amersham Pharmacia Biotech, Piscataway, N.J.

The assay of the present invention was performed as described in Example 1, except for the addition of known PARP inhibitors to the autoribosylation buffer. The inhibitors were first dissolved in DMSO and then serially diluted in DMSO to create stock solutions which were added to the autoribosylation buffer to achieve the desired range of final concentrations of the inhibitors in the nanomolar to millimolar range.

The PARP inhibitors used in this study included 3-aminobenzamide (3-AB, Sigma Aldrich, Catalog #A-0783), 8-hydroxy-2-methyl-quinazolin-4-[3H]-one (Compound A, U.S. Pat. No. 5,756,510), and 8-carboximido-napthalene-1-carboxylic acid (Compound B, WO 99/11622). The IC₅₀ values generated by the assay of the present invention (Biotin-Avidin PARP assay) were compared to the values obtained using the [³²P]-based assay as well as values reported in the literature, as shown in Table 1.

**Table 1**

| Compound | Biotin Avidin PARP (IC₅₀) | [³²P] PARP Assay (IC₅₀) | IC₅₀ cited in the Literature* |
|---|---|---|---|
| | | | |
| 3-AB | 27.8 µM | 10.8 µM | 10-20 µM |
| | | | |
| Compound A | 460 nM | 318 nM | 400 nM |
| | | | |
| Compound B | 329 nM | 334 nM | 250 nM |

| | | | |
|---|---|---|---|
| * Reported IC₅₀ values for 3-AB (Decker et al. (2000) *Clin. Cancer Res.* **5**: 1169-1172; Rankin et al. (1989) *J. Biol. Chem.* **264:** 4312-4317; and Banasik et al., (1992) *J. Biol. Chem.* **267:** 1569-1575), Compound A (US Pat. No. 5,756,510), and Compound B (WO 99/11622). | | | |

Those skilled in the art will recognize, or be able to ascertain, using no more than routine experimentation, many equivalents to the specific embodiments of the invention described herein. Such equivalents are intended to be encompassed by the following claims.

## Claims

1. A method to assay PARP activity, comprising
(a) contacting an immobilized PARP with NAD under conditions that allow PARP auto-ribosylation, wherein said NAD is biotinylated or avidin-conjugated;
(b) contacting the auto-ribosylated PARP with a detectable marker, wherein said detectable marker is avidin-conjugated in the case where the NAD of (a) is biotinylated, or wherein said detectable marker is biotinylated in the case where the NAD of (a) is avidin-conjugated, thereby forming a complex between the auto-ribosylated PARP and the detectable marker; and
(c) measuring the amount of detectable marker complexed to auto-ribosylated PARP, wherein the amount of detectable marker is indicative of the amount of PARP activity.

2. The method of claim 1, wherein said PARP is immobilized on a multiwell plate.

3. The method according to either claim 1 or claim 2, wherein said method is conducted at 4°C.

4. A method to assay PARP activity, comprising
(a) contacting an immobilized PARP with biotinylated NAD under conditions that allow PARP auto-ribosylation;
(b) contacting the auto-ribosylated PARP with an avidin-conjugated alkaline phosphatase, thereby forming a complex between the auto-ribosylated PARP and the avidin-conjugated alkaline phosphatase; and
(c) measuring the amount of alkaline phosphatase complexed to the auto-ribosylated PARP, wherein the amount is indicative of the amount of PARP activity.

5. A method to identify a modulator of PARP activity, comprising:
(a) contacting an immobilized PARP with NAD in the presence of a test agent under conditions that allow PARP autoribosylation, wherein said NAD is biotinylated or avidin-conjugated;
(b) contacting the auto-ribosylated PARP with a detectable marker, wherein said detectable marker is avidin-conjugated in the case where the NAD of (a) is biotinylated, or wherein said detectable marker is biotinylated in the case where the NAD of (a) is avidin-conjugated, thereby forming a complex between the auto-ribosylated PARP and the detectable marker;
(c) measuring the amount of detectable marker complexed to the autoribosylated PARP; and
(d) comparing the amount of detectable marker in step (c) to the amount of detectable marker in a control reaction executed without the test agent;
wherein an altered amount of detectable marker in the presence of the test agent relative to the amount of detectable marker in the control reaction indicates that the test agent is a modulator of PARP.

6. The method of claim 5, wherein said PARP is immobilized on a multiwell plate.

7. The method according to either claim 5 or claim 6, wherein said method is conducted at 4°C.

8. A method to identify a modulator of PARP activity, comprising:
(a) contacting an immobilized PARP with biotinylated NAD in the presence of a test agent under conditions that allow PARP auto-ribosylation;
(b) contacting the auto-ribosylated PARP with an avidin-conjugated alkaline phosphatase, thereby forming a complex between the auto-ribosylated PARP and the avidin-conjugated alkaline phosphatase;
(c) measuring the amount of alkaline phosphatase complexed to the autoribosylated PARP; and
(d) comparing the amount of alkaline phosphatase in step (c) to the amount of alkaline phosphatase in a control reaction executed without the test agent;
wherein an altered amount of alkaline phosphatase in the presence of the test agent relative to the amount of alkaline phosphatase in the control reaction indicates that the test agent is a modulator of PARP.

9. A kit comprising:
(a) PARP immobilized on a solid support;
(b) biotinylated NAD or avidin-conjugated NAD; and
(c) an avidin-conjugated detectable marker in the case where the NAD of (b) is biotinylated, or a biotinylated detectable marker in the case where the NAD of (b) is avidin-conjugated.

10. A kit comprising:
(a) PARP immobilized on a solid support;
(b) biotinylated NAD; and
(c) avidin-conjugated alkaline phosphatase.
